# EUROPÄISCHE PATENTSCHRIFT

(11) **EP 1 915 135 B1**
(45) Veröffentlichungstag und Bekanntmachung des Hinweises auf die Patenterteilung: **24.04.2013**
(21) Anmeldenummer: 06776684.0
(22) Anmeldetag: 08.08.2006
(51) Int. Cl.: A61K 9/16, B01J 2/16

(54) **Verfahren zur Herstellung von kugelförmigen Teilchen aus einer pharmazeutischen Substanz**
Method of manufacturing spheric particles on the basis of pharmaceutical substances
Procédé pour produire les particules sphériques à base de substances pharmaceutiques

(30) Priorität: 09.08.2005 DE 102005037630
(43) Veröffentlichungstag der Anmeldung: 30.04.2008
(73) Patentinhaber: GLATT GMBH, 79589 Binzen (DE)
(72) Erfinder: JACOB, Michael, 99427 Weimar (DE); GRAVE, Annette, 79540 Lörrach (DE); NOWAK, Reinhard, 79589 Binzen (DE)
(74) Vertreter: Henkel, Breuer & Partner
(86) Internationale Anmeldenummer: PCT/EP2006/007848
(87) Internationale Veröffentlichungsnummer: WO 2007/017254

(56) Entgegenhaltungen:
- EP-A2- 0 362 731
- US-A- 3 231 413
- US-A- 4 086 346
- US-A- 4 092 089

## Beschreibung

### Gebiet der Erfindung

Die Erfindung betrifft ein Verfahren zur Herstellung von Teilchen aus einer Schmelze, die Teilchen selbst sowie deren Verwendung. Die Teilchen werden aus pharmazeutischen Substanzen (Arznei- und/oder Hilfsstoffen) gebildet.

### Hintergrund der Erfindung

Feinteilige Formen von pharmazeutischen Substanzen, wie feine Pulver, sind häufig mit verarbeitungstechnischen Nachteilen behaftet. Um solche Nachteile zu vermeiden, werden häufig gröbere Teilchen (Granulate) bereitgestellt.

Solche Teilchen aus pharmazeutischen Substanzen können zur Herstellung von Darreichungsformen verwendet werden, indem sie beispielsweise allein oder zusammen mit weiteren Komponenten zu Tabletten verpresst werden. Arzneistoffhaltige Teilchen können darüber hinaus eingefüllt in Kapseln oder in Form eines Pulvers für eine Suspension oder Lösung verwendet werden.

Zur Herstellung von Teilchen sind eine Reihe von Verfahren bekannt. Bei einem Teil der Verfahren werden Lösungsmittel eingesetzt. Dies ist von Nachteil, da die Lösungsmittel im Verlauf des Verfahrens wieder entfernt werden müssen. Auch geringe zurückbleibende Lösungsmittelmengen können die Produktqualität beeinträchtigen. Organische Lösungsmittel sind darüber hinaus unter dem Gesichtspunkt der Arbeitssicherheit und des Umweltschutzes unerwünscht.

Alternativ sind Schmelzerstarrungsgranulate (Schmelzgranulate) bekannt. Sie werden durch Schmelzen und Schockerstarren, durch Ausgießen und Zerkleinern oder durch Sprüherstarren in Sprühtürmen hergestellt. Diese bekannten Verfahren sind jedoch mit Nachteilen behaftet.

Verfahren, bei denen eine erstarrte Schmelze zerkleinert wird, sind recht aufwendig, da sie getrennt Stufen des Schmelzens, Erstarrens und Zerkleinerns erfordern, für die jeweils verschiedenartige Vorrichtungen benötigt werden. Zudem ist es recht schwierig, ein teilchenförmiges Material mit einer Teilchengrößeverteilung zu erhalten. Überdies sind die durch Zerkleinern erhaltenen Teilchen unregelmäßig geformt, was die weitere Handhabung erschwert.

Auch die Herstellung eines Schmelzgranulates durch Erstarren einer Schmelze in einem Sprühturm ist mit Nachteilen behaftet. Typischerweise wird ein relativ großer Anteil an Material mit unerwünschter Teilchengröße gebildet, das abgetrennt und erneut aufgeschmolzen werden muss. Die erhaltenen Teilchen sind in der Regel nicht gleichmäßig kugelförmig, was ihre Handhabungseigenschaften beeinträchtigt.

Ein Verfahren der letztgenannten Art ist in EP 0 362 731 beschrieben. Zur Durchführung des Verfahrens wird eine Arzneimittelwirkstoff-Schmelze in einem Sprühkühlturm zerstäubt, um Arzneimittelwirkstoff-Partikel herzustellen. Beispielhaft wird Ibuprofen als Wirkstoff eingesetzt. Die Partikel werden durch Abkühlen von Tröpfchen einer Schmelze erhalten, die in einem Sprühkühlturm in Gegenwart von Kristallisationskeimen zerstäubt und mit einem Kühlgas in Kontakt gebracht wird. Aus dem gebildeten Pulver werden gewünschte Partikel mit Hilfe eines Siebs abgetrennt.

Die Herstellung von Arzneistoffpartikeln, speziell von Ibuprofen-Partikeln, sowie deren Verwendung zur Herstellung bestimmter Darreichungsformen, ist auch Gegenstand einer Reihe weiterer Patente und Patentanmeldungen.

EP 0 362 728 A2 betrifft ein Verfahren zur Gewinnung von Ibuprofen für die Direkttablettierung. Dabei wird eine Ibuprofen-Schmelze auf einem Kontaktkühlapparat verfestigt und anschließend zerkleinert. Da die Herstellung der Partikel in zwei Stufen erfolgt, ist das Verfahren recht aufwendig. Zudem sind die Partikel durch den Zerkleinerungsprozess unregelmäßig geformt.

US 6 322 816 betrifft analgetische Präparate mit schneller Wirkstofffreisetzung. Als Wirkstoff kommt insbesondere Ibuprofen in Betracht. Der Wirkstoff liegt in einer speziellen Adjuvansmatrix vor.

US 2003/0203026 betrifft therapeutische Mittel, insbesondere eine gepresste Tablette. Darin enthalten ist eine granuläre Komponente, die eine Mehrzahl von verfestigten Schmelzkörnern aus einem nicht steoidalen entzündungshemmenden Arzneistoff, der einen Schmelzpunkt im Bereich von 30 bis 200 °C hat, und einem darin gleichmäßig verteiltes Zerfallsmittel enthält. Ibuprofen ist ein bevorzugter Wirkstoff. Die Schmelze kann durch Kühlen verfestigt und dann zerkleinert werden. Alternativ kann die Schmelze durch eine Düse versprüht werden, um eine Verfestigung des Materials zu ermöglichen, das dann aufgefangen wird.

US 2005/0003000 betrifft ein Verfahren zur Bildung von Ibuprofen-Feststoffen, wobei einer Lösung des Ibuprofens Additive zugesetzt werden, die später wieder entfernt werden.

WO 02/07706 betrifft ein Verfahren zum Überziehen von festen Partikeln, beispielsweise Ibuprofen-Partikeln. Das Dokument beschäftigt sich nicht mit der Herstellung dieser als Ausgangsmaterial eingesetzten Partikel.

WO 94/10993 betrifft pharmazeutische Formulierungen von Ibuprofen. Es wird unter anderem die Herstellung einer partikulären Darreichungsform beschrieben, wobei die Zugabe einer wässrigen Lösung eines Bindemittels zu Ibuprofen vorgesehen ist.

US 5 320 855 betrifft Kautabletten, die aus Körnchen eines Medikamentes hergestellt werden. Die Körnchen wiederum werden durch Rotogranulation eines Gemisches aus einem Arzneistoff, wie Ibuprofen, und Hilfsstoffen hergestellt und mit bestimmten Überzügen versehen.

EP 0 290 168 betrifft eine Ibuprofen-Tablette mit verzögerter Freisetzung. Vorgesehen ist insbesondere eine Ibuprofen enthaltende Matrix, die durch Granulation von Ibuprofen im Gemisch mit pulverförmigen Hilfsstoffen unter Verwendung einer bestimmten Lösung als Granulationsflüssigkeit erhalten wird.

EP 0 241 126 betrifft eine feste pharmazeutische Zusammensetzung, die Körner umfasst, die im Wesentlichen aus einem Aggregat von Ibuprofen-Kristallen bestehen. Allgemein umfasst das Verfahren das Kompaktieren von kristallinem Ibuprofen, um eine Aggregation des kristallinen Ibuprofens unter Bildung eines Aggregatmaterials zu bewirken, die Zerkleinerung des Aggregatmaterials und die Selektion von Körnern mit der gewünschten Größe. Beispielsweise kann zur Herstellung kristallines Ibuprofen mit einem Lösungsmittel benetzt werden. Es erfolgt dann eine Extrusion. Die dabei erhaltenen Körner werden getrocknet.

EP 0 230 322 betrifft eine pharmazeutische Zusammensetzung mit verzögerter Freisetzung des Wirkstoffes. Dabei kommen Zuckeresther höherer Fettsäuren zum Einsatz. Das Herstellungsverfahren umfasst das Mischen und Granulieren von Komponenten.

EP 0 250 648 betrifft ein pharmazeutisches Präparat zur verzögerten Freigabe von Ibuprofen. Das Präparat liegt in Form von Tabletten vor, die den Wirkstoff in Mikrosphären enthalten. Die Herstellung erfolgt, indem man das Ibuprofen und ein Bindemittel zu einer homogenen Mischung vermischt und mit Wasser befeuchtet. Durch Extrusion wird die Mischung dann zu Mikrosphären geformt, aus denen schließlich Tabletten hergestellt werden.

WO 96/31197 betrifft homogene Gemische von niedrigschmelzenden Arzneistoffen und Additiven für die kontrollierte Freisetzung. Ein Verfahren zur Herstellung einer derartigen Formulierung umfasst das Schmelzen eines Arzneistoffes und eines Additivs bei einer Temperatur unterhalb von 150 °C, das Mischen des Arzneistoffes und des Additivs unter Bildung eines homogenen Gemisches sowie schließlich das Härten des homogenen Gemisches unter Bildung eines Arzneistoff-Additiv-Verbundmaterials. Es ist vorgesehen, die geschmolzene Masse in Kapseln einzufüllen, wo sie dann beim Abkühlen aushärtet.

Der Stand der Technik enthält weiterhin Vorschläge, Granulate unter Verwendung von Strahlschichtapparaten zu erzeugen. So ist aus DE 103 22 062 A1 bekannt, Granulate verschiedenartiger Materialien durch Aufbringen von Flüssigkeiten in eine Feststoffströmung eines Strahlschichtapparates herzustellen. Die genannte Anmeldung beschäftigt sich jedoch weder mit den Besonderheiten pharmazeutischer Substanzen noch mit den Bedingungen, die bei der Herstellung von Teilchen aus einer Schmelze beachtet werden müssen.

DE 100 04 939 C1 betrifft eine steuerbare Gasanströmeinrichtung für Strahlschichtapparate. Mit der Herstellung von Schmelzgranulaten beschäftigt sich die Patentschrift nicht.

WO 2004/108911 A2 betrifft Herstellungsverfahren für Enzymgranulate sowie derartige Granulate. Zu der Herstellung wird ein Strahlschichtapparat eingesetzt. Mit Schmelzgranulaten befasst sich die Anmeldung nicht.

In EP0362731 wird die Arzneimittelwirkstoff-Schmelze (Ibuprofen) später einer Sprühkühlung unterworfen, wodurch Wirkstoffpartikel entstehen. Dann werden die Partikel zu Tabletten verpresst. US4086346 legt einen Prozess zur Herstellung von kugelförmigen Phenacetingranulaten im Schmelz-Sprüh-Verfahren offen, wobei die Phenacetinpartikel durch Einströmen von inertem Gas (Luft, Stickstoff, Kohlendioxid) gehärtet werden. In US3231413 wird von einem Prozess der Granulierung flüssigen Materials (durch Schmelzen) zu Partikeln von 2 Millimetern im Durchmesser infolge Versprühen und Verbringen von Ausgangsmaterial in eine verflüssigte Phase berichtet. US4092089 stellt einen Apparat zum Versprühen von geschmolzenem Phenacetin vor.

### Aufgaben und Kurzdarstellung der Erfindung

Eine Aufgabe der vorliegenden Erfindung besteht darin, ein Verfahren anzugeben, mit dem vorzugsweise kugelförmige Teilchen von pharmazeutischen Substanzen (Arznei- und/oder Hilfsstoffen) aus einer Schmelze hergestellt werden können. Die Nachteile herkömmlicher Verfahren sollen dabei vermieden werden können. Eine weitere Aufgabe besteht darin, vorzugsweise kugelförmige Teilchen sowie Kollektive solcher Teilchen bereitzustellen und deren Verwendung für die Herstellung pharmazeutischer Darreichungsformen zu ermöglichen.

Erfindungsgemäß wurde festgestellt, dass der Aufbau im Wesentlichen kugelförmiger Teilchen aus einzelnen Tröpfchen einer Schmelze möglich ist, wenn die Tröpfchen mit Hilfe eines geeignet temperierten Prozessgases so geführt werden, dass Teilchen aus bereits erstarrter Schmelze erneut mit Tröpfchen in Kontakt kommen können. Im Unterschied zur Herstellung von Teilchen aus einer Schmelze in einem herkömmlichen Sprühturm werden die sich bildenden Teilchen in einem geeigneten Prozessraum solange im Kreis geführt, bis sie durch wiederholte Anlagerung von Tröpfchen der Schmelze die gewünschte Größe erreicht haben.

Dementsprechend wird erfindungsgemäß ein Verfahren zur Herstellung von Teilchen mit einem Länge-Breite-Verhältnis von weniger als 1,4 wie dargestollt in Anspruch 1 aus einer pharmazeutischen Substanz bereitgestellt, das folgende Stufen umfasst:
(a) Bereitstellung einer Schmelze der pharmazeutischen Substanz;
(b) Erzeugen von Tröpfchen der Schmelze durch Einsprühen in einen Prozessraum;
(c) wiederholtes Vorbeiführen von festen Teilchen an eingesprühten Tröpfchen in dem Prozessraum mit Hilfe eines definiert geführten Prozessgasstrahls, dessen Temperatur in Abhängigkeit vom Erstarrungspunkt der Schmelze festgelegt ist, so dass zumindest ein Teil der Tröpfchen mit Teilchen in Kontakt kommt und auf diesen erstarrt;
(d) Entnehmen von Teilchen aus dem Prozessraum in Abhängigkeit von der Teilchengröße.

Erfindungsgemäß werden auch Teilchen aus einer pharmazeutischen Substanz bereitgestellt, wobei die Teilchen eine mittlere Teilchengröße von 0,1 bis 3 mm und ein Länge-Breite-Verhältnis von weniger als 1,4 (und wie ferner dargestellt in Anspruch 1) aufweisen und aus einer Schmelze der pharmazeutischen Substanz hergestellt sind.

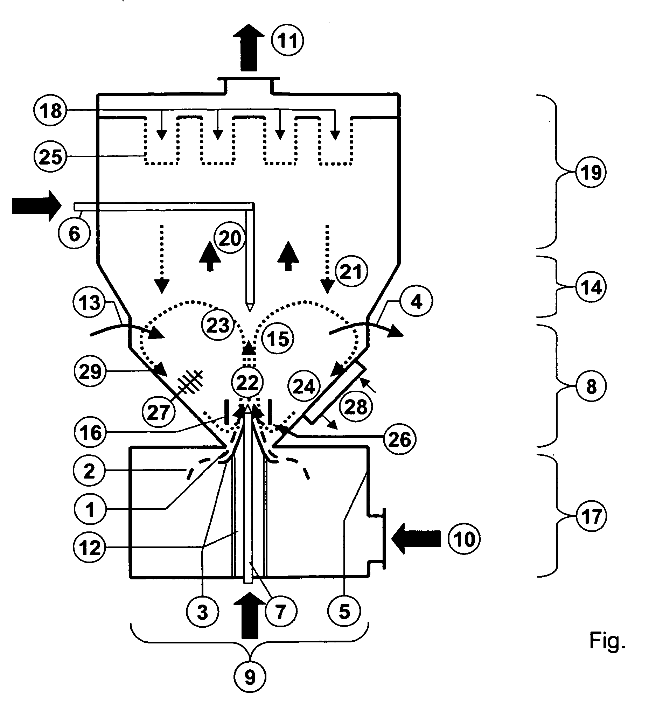

### Kurze Beschreibung der Figur

Die Erfindung wird nachstehend unter Bezugnahme auf eine Figur näher erläutert. In dieser Figur ist schematisch eine Anlage zur Durchführung des erfindungsgemäßen Verfahrens dargestellt.

### Ausführliche Darstellung der Erfindung

### Definitionen

Nachstehend werden einige technische Begriffe erläutert, die in der Beschreibung und in den Ansprüchen verwendet werden.

Eine Schmelze wird durch Schmelzen eines Stoffes unter Erwärmen auf eine Temperatur, die typischerweise im Bereich von 30 °C bis 300 °C liegt, erzeugt. Vorzugsweise wird die Schmelze durch vollständiges Aufschmelzen eines Stoffes oder Stoffgemisches erhalten, so dass eine homogene Phase vorliegt. Alternativ können feste Stoffe in der Schmelze dispergiert sein. Sofern nichts anderes angegeben ist, wird der Begriff Schmelze hier in diesem weiteren Sinn verstanden.

Der Ausdruck "pharmazeutische Substanz" soll Arzneistoffe, pharmazeutische Hilfsstoffe sowie Gemische solcher Komponenten bezeichnen. Gemäß einer bevorzugten Ausführungsform handelt es sich bei der pharmazeutischen Substanz um einen Arzneistoff.

Da erfindungsgemäß Teilchen aus pharmazeutischen Substanzen durch Schmelzgranulation hergestellt werden sollen, ist es weiterhin erforderlich, dass die Substanzen ohne wesentliche Zersetzung, das heißt ohne Beeinträchtigung ihrer pharmazeutischen Verwendung, aufgeschmolzen werden können. Substanzen, die beim Schmelzen einer Zersetzung unterliegen, können erfindungsgemäß nicht verarbeitet werden, sofern nicht der Schmelzpunkt durch geeignete Zusätze so herabgesetzt werden kann, dass das Schmelzen und Verarbeiten möglich sind. Der Fachmann kann durch Tests feststellen, ob nach dem Schmelzen und Erstarren noch eine ausreichende Qualität gegeben ist. Tests zur Sicherstellung der Qualität zahlreicher pharmazeutischer Substanzen sind in den entsprechenden Arzneibüchern festgelegt.

Beispiele für Arzneistoffe, die durch Schmelzgranulation verarbeitet werden können, sind Divalproexnatrium, Ibuprofen, Ramipril, Dibenzylin, Erythrityltetranitrat, Isosorbiddinitrat, Methosuximid, Ketoprofen, Gemfibrozil, Paroxetinhydrochlorid und Trimipraminmaleat. Ein erfindungsgemäß bevorzugter Arzneistoff ist Ibuprofen.

Beispiele für pharmazeutische Hilfsstoffen sind niedrigschmelzende Binder, zum Beispiel Gelatine und Stärkemittel, sowie niedrigschmelzende unlösliche Polymere.

Erfindungsgemäß wird ein Teilchen als kugelförmig bezeichnet, wenn das Länge-Breite-Verhältnis (das heißt das Verhältnis der Länge (größte Abmessung) des Teilchens, dividiert durch die Breite (kleinste Abmessung), die in einem Winkel von 90° in Bezug auf die Länge festgestellt wird) weniger als 1,4 beträgt. Vorzugsweise beträgt das Länge-Breite-Verhältnis eines kugelförmigen Teilchens weniger als 1,3, stärker bevorzugt weniger als 1,2, noch stärker bevorzugt weniger als 1,1 und insbesondere weniger als 1,05.

Die Teilchen sind darüber hinaus durch ihre Größe charakterisiert. Die Teilchengrößeverteilung kann durch Siebanalyse bestimmt werden. Soweit nichts anderes angegeben ist, bezieht sich die Teilchengröße auf das Gewichtsmittel.

Gegenstand der vorliegenden Erfindung ist auch ein Produkt, das eine Mehrzahl von Teilchen umfasst. Ein derartiges Produkt umfasst ein Kollektiv von Teilchen, typischerweise 50 oder mehr, vorzugsweise 100 oder mehr Teilchen. Ein erfindungsgemäßes Produkt umfasst überwiegend Teilchen, die die erfindungsgemäßen Teilchenkriterien erfüllen. Vorzugsweise weisen mindestens 90 %, insbesondere mindestens 95 % und ganz besonders bevorzugt mindestens 98 % der Teilchen ein Länge-Breite-Verhältnis von weniger als 1,4, vorzugsweise weniger als 1,3, stärker bevorzugt weniger als 1,2, noch stärker bevorzugt weniger als 1,1 und insbesondere weniger als 1,05 auf.

Erfindungsgemäß wird ein Prozessgasstrahl genutzt, um feste Teilchen wiederholt an eingesprühten Tröpfchen vorbeizuführen. Bei dem Prozessgas kann es sich beispielsweise um Luft oder ein Inertgas, wie Stickstoff, Kohlendioxid oder ein Edelgas handeln.

### Bevorzugte Ausführungsformen

Wie bereits erläutert, wurde erfindungsgemäß festgestellt, dass es möglich ist, kugelförmige Teilchen aus einzelnen Tröpfchen einer Schmelze herzustellen. Die Teilchen weisen vorzugsweise einen kompakten Aufbau auf. Weiterhin ist es bevorzugt, wenn die Teilchen eine homogene Oberflächenstruktur haben. Dabei ist es wesentlich, dass der Aufbau kugelförmiger Teilchen ermöglicht wird, indem vorgelegte oder aus der Schmelze gebildete Teilchen wiederholt mit Tröpfchen der Schmelze in Kontakt kommen, so dass kugelförmige Teilchen einer gewünschten Größe aufgebaut werden können. Dazu werden die Teilchen innerhalb eines Prozessraumes mit Hilfe eines definiert geführten Prozessgasstrahls bewegt. Teilchen, die eine gewünschte Größe erreicht haben, können den Prozessraum verlassen.

Der Prozessgasstrahl ist sowohl für den Stofftransport als auch für den Wärmetransport wesentlich. Erfindungsgemäß wird durch Wahl der Temperatur des Prozessgasstrahls in Abhängigkeit vom Erstarrungspunkt der Schmelze erreicht, dass es zu einem Kontakt der eingesprühten Tröpfchen mit bereits verfestigten Teilchen unter Ausbildung weitgehend kugelförmiger Teilchen kommt. Insbesondere wird für solche Temperaturbedingungen im Prozessraum gesorgt, dass die Erstarrung ausreichend verzögert ist, um eine Benetzung der bereits festen Teilchen mit den eingesprühten Tröpfchen der Schmelze und die Ausbildung kugelförmiger Strukturen zu ermöglichen. Andererseits wird erfindungsgemäß weitgehend vermieden, dass Teilchen mit einer flüssigen Oberfläche miteinander in Kontakt treten und verkleben.

Entsprechend weist der Prozessgasstrahl eine Temperatur auf, die unterhalb des Erstarrungsproduktes der Schmelze liegt. Andererseits darf die Temperatur des Prozessgasstrahls kein sofortiges Erstarren von Tröpfchen erlauben, die in den Prozessraum eingesprüht werden. Vorzugsweise liegt die Temperatur des Prozessgasstrahls 10 °C bis 40 °C unterhalb des Erstarrungspunktes der Schmelze.

Erfindungsgemäß ist es bevorzugt, Tröpfchen der Schmelze und feste Teilchen in einer Strahlschicht miteinander in Kontakt zu bringen. Unter Strahlschicht wird verstanden, dass die vollständig fluidisierten festen Teilchen sich in einer zeitlich stabilen geschlossenen Feststoffströmung befinden. Die Strahlschicht wird mit Hilfe des definiert geführten Prozessgasstrahls erzeugt. Innerhalb der Strahlschicht sind drei Fluidisationszustände oder-zonen zu unterscheiden. In einer ersten Zone oder Auswurfzone erfolgt die Beschleunigung der festen Teilchen unter Einwirkung des definiert geführten Prozessgasstrahls, wobei sich die Teilchen in dieser Zone in der Strömungsrichtung des Prozessgasstrahls bewegen. Typischerweise wird der Prozessgasstrahl senkrecht nach oben geführt. Entsprechend herrscht in der Auswurfzone der Strahlschicht eine senkrecht nach oben gerichtete Strömung vor. In einer anschließenden zweiten Zone oder Fontänenzone ändern die Teilchen ihre Strömungsrichtung. Es herrscht eine Querströmung vor. Schließlich gelangen die Teilchen in eine dritte Zone oder Rücklaufzone. Dort weisen die Teilchen dann eine entgegengerichtete Bewegung auf, bis sie schließlich wieder in den Einfluss des definiert geführten Gasstromes gelangen und in der ersten Zone erneut von diesem mitgeführt werden. In der Rücklaufzone bewegen sich die Teilchen typischerweise unter dem Einfluss der Schwerkraft.

Das Versprühen der Schmelze kann durch Zwei- und Mehrstoffdüsen erfolgen. Weiterhin ist es möglich, das Versprühen durch Druckdüsen zu bewirken. Alternativ kann ein Vertropfen durch Rotationszerstäuber, Strahlschneider, Ultraschallzertropfer und andere dem Fachmann bekannte Vorrichtungen erfolgen.

Erfindungsgemäß ist es möglich, durch Einsprühen von Tröpfchen einer Schmelze in den Prozessraum und Erstarrenlassen dieser Tröpfchen Keime aus festen Teilchen zu bilden, die dann mit weiteren Tröpfchen in Kontakt gebracht werden, um Teilchen der gewünschten Größe zu bilden. Alternativ oder zusätzlich können bei dem Verfahren feste Teilchen von außen zugeführt werden. Beispielsweise können aus dem Prozess entnommene zu kleine Teilchen in den Prozessraum als Keimmaterial zurückgeführt werden. Ebenso können zu große aus dem Prozess entnommene Teilchen oder Agglomerate von Teilchen durch ein beliebiges Zerkleinerungsaggregat zerkleinert und in den Prozessraum als Keimmaterial zurückgeführt werden. Es ist auch möglich, Teilchen anderer Zusammensetzungen als der der Schmelze zuzuführen. Auf diese Weise ist eine Schmelzeinbettung der zugeführten Teilchen möglich.

Die nach dem erfindungsgemäßen Verfahren gebildeten Teilchen werden dem Prozessraum entnommen. Der Materialaustrag des Fertigproduktes aus dem Prozessraum oder ein Materialtransport in einen weiteren nachgeschalteten Prozessraum kann im Bereich des Übergangs der Querströmung zu der nach unten gerichteten Feststoffströmung erfolgen. Gemäß einer Ausführungsform werden die aus dem Prozessraum ausgetragenen Teilchen nicht klassiert. Gemäß einer anderen Ausführungsform werden die aus dem Prozessraum ausgetragenen Teilchen klassiert durch ein oder mehrere Sichtapparate entnommen.

Das erfindungsgemäße Verfahren kann beispielsweise mit Hilfe einer Vorrichtung durchgeführt werden, wie sie in DE 103 22 062 A1 beschrieben ist. Der Inhalt dieser Anmeldung wird durch Verweis zum Gegenstand der vorliegenden Anmeldung gemacht.

Vorzugsweise wird das erfindungsgemäße Verfahren unter Verwendung einer Vorrichtung, wie sie in der beigefügten Figur gezeigt ist, durchgeführt. Dies wird nachstehend ausführlich erläutert.

Die zur Verfestigung der herzustellenden Teilchen erforderliche Menge an Prozessgas 10 (in der Regel erwärmte Luft) wird einer Zuluftkammer 17 mit rechteckigem Querschnitt 9 und begrenzenden Seitenwänden 5 zugeführt. In der Zuluftkammer 17 verteilt sich das Prozessgas 10 und tritt über Spaltöffnungen 1 in den Prozessraum 8 in Form von Gasstrahlen 2 ein. Der vorzugsweise horizontal in den Spalt 1 eintretende Prozessgasstrom wird durch das Umlenkteil 3 vorzugsweise nach oben in den Prozessraum 8 hinein umgelenkt und strömt als eine Art Freistrahl in den Apparat hinein. Im Weiteren kann sich der Apparatequerschnitt optional in der Expansionszone 14 vergrößern, so dass sich die Geschwindigkeit der Prozessgasströmung nach oben hin stetig verringert. Das Gas verlässt den Apparat als Abgas 11 oberhalb der Expansionszone 14 über das Abluftteil 19, in das optional ein Entstaubungssystem (z.B. Filterpatronen oder Textilfilterelemente) integriert werden kann.

Im Prozessraum 8 befindet sich eine Menge an Teilchen, die durch den Prozessgasstrahl nach oben hin mitgerissen werden. Im oberen Bereich des Prozessraumes 8 sowie in der darüber befindlichen Expansionszone 14 nimmt die Gasgeschwindigkeit ab, so dass die aufwärts strömenden Teilchen seitlich aus dem Gasstrahl 23 heraustreten und in den Prozessraum 8 zurückfallen. Der Prozessraum 8 wird im unteren Bereich von geneigten Seitenwänden 29 begrenzt. Bedingt durch diese Seitenneigung werden die Teilchen unter Wirkung der Schwerkraft über die Rücklaufzone 24 in Richtung des Gaseintrittsspaltes 1 befördert, wo sie anschließend wieder vom Prozessgas in den Prozessraum 8 mitgerissen werden.

Durch diesen Mechanismus bildet sich eine sehr gleichförmige Feststoffzirkulation 15 bestehend aus einer Aufwärtsströmung und einem Rücklauf in Richtung des Prozessgaseintrittes. Dadurch liegt auch bei sehr geringen Mengen an Teilchen im Prozessraum 8 in der Kernzone oberhalb des Umlenkteiles 3 eine hohe Partikeldichte vor. In diesem Bereich werden ein oder mehrere Sprühdüsen 7 angeordnet, die gleichgerichtet zum Prozessgasstrahl nach oben sprühen und zum Einbringen der Schmelze dienen.

Durch die hohe Partikelbeladung in der Kernzone ergeben sich in der Bedüsungszone 22 sehr vorteilhafte Bedingungen für den Wärme- und Stoffübergang. Weiterhin wird erreicht, dass sich die Schmelze weitestgehend an den Teilchen abscheidet und diese somit gleichmäßig an den Partikeloberflächen benetzt werden. Das gleichmäßige Benetzen bei gleichzeitiger hoher Feststoffzirkulation zwischen Bedüsungsbereich und Rücklaufzone 24 bewirkt, dass ein sehr gleichmäßiger Flüssigkeitsfilm gebildet wird. Durch den Erstarrungsprozess verfestigt sich die Schmelze und der Feststoff verbleibt auf der Teilchenoberfläche. Dadurch wachsen die Granulate sehr gleichförmig und homogen, was zu einer sehr engen Korngrößenverteilung und einer homogenen Partikelstruktur führt.

Das Prozessgas kann einen Teil der Partikeln sowie Feingut und Stäube als feststoffbeladene Abluft 20 aus dem Prozessraum 8 ausgetragen. Zur Abscheidung dieser Teilchen können das im Abluftteil 19 optional integrierte Filtersystem oder dem Apparat nachgeschaltete Entstaubungsanlagen verwendet werden. Im Falle einer integrierten Entstaubungsanlage 25 können beispielsweise Druckluftimpulse 18 genutzt werden, um die zurückgehaltenen Partikeln als abgetrennten Feststoff 21 in den Prozessraum 8 zurückzuführen.

Im Vergleich zu Wirbelschichtapparaten mit integrierten Filteranlagen wird die Staubrückführung dadurch erleichtert, dass die aufwärts gerichtete Prozessgasströmung im Wesentlichen örtlich begrenzt ist und somit die zurückzuführenden Teilchen außerhalb des Gasstrahles sicher absinken können. Durch die Sogwirkung in der Nähe des Gaseintrittsspaltes 1 wird dieser Mechanismus zusätzlich gefördert. Alternativ können von der Abluft abgeschiedene Teilchen in den Prozessraum 8 zurückgeführt werden. Dazu können im unteren Bereich der geneigten Seitenwände 29 verschiedenartige Zuführungen 26 angeordnet sein. Bedingt durch die hohe Geschwindigkeit des Prozessgasstrahls in der Nähe des Gaseintrittsspaltes 1 werden die feinen Partikeln angesaugt und der Bedüsungszone 22 zugeführt, wo diese mit Schmelze benetzt werden und am Wachstumsprozess teilnehmen.

Optional eingebaute Leitbleche 16 stützen den Gasstrahl, verstärken den Sogeffekt und verbessern die Zuführung der Feststoffe in der Bedüsungszone 22 hinein. Eventuell auftretende Agglomerationseffekte werden minimiert, da im Bedüsungsbereich sehr hohe Strömungsgeschwindigkeiten und somit höhere Trennkräfte als in Wirbelschichten auftreten. Dadurch werden Teilchen separiert und wachsen zu Granulaten mit Kugelgestalt.

Das Strömungsprofil des Prozessgases im Prozessraum 8 bewirkt weiterhin, dass von der optional integrierten Filteranlage in den Prozessraum zurückgeführte Feinpartikel nicht in die Bedüsungszone 22 zurückfallen. Dadurch wird das Verkleben von Feinpartikeln und daraus folgende Agglomeratbildungsprozesse unterbunden.

Zur kontinuierlichen Prozessführung kann der Apparat optional mit unterschiedlichen Eintragsystemen 13 für Feststoffe ausgerüstet werden. Dadurch können beispielsweise Partikel dem Prozess zugeführt werden, die durch Zerkleinerung von beispielsweise (zu großen) Granulaten gewonnen werden können oder/und aus zu kleinen Granulaten bestehen. Diese Teilchen dienen dann als Granulationskeime oder als Startfüllung zur Verkürzung der Inbetriebnahmezeit. Außerdem können hier Additive in fester Form in den Prozess eingeschleust werden, die in die Granulate eingebettet werden sollen.

Weiterhin kann der Apparat mit Austragsorganen 4 versehen werden, um Partikel aus dem Prozessraum 8 entnehmen zu können. Dies kann beispielsweise durch einen Überlauf oder durch ein volumetrisches Austragsorgan (z.B. eine Zellenradschleuse) oder auch durch einen Schwerkraftsichter (z.B. einen mit Sichtgas beaufschlagten Zick-Zack-Sichter oder einen Steigrohrsichter) erfolgen.

Optional können mechanische Aggregate 27 im Prozessraum 8, jedoch vorzugsweise im Bereich der Rücklaufzone 24 an den geneigten Wänden angebracht werden, um durch Zerkleinerung ausreichend Feinmaterial als Keime für den Granulatbildungsprozess zu erzeugen. Weiterhin kann die Rücklaufzone 24 optional zur Positionierung von Beheizungen oder anderen Wärmeübertragungseinrichtungen 28 genutzt werden. Beispielsweise kann die Apparatewand doppelwandig ausgeführt sein, um diese beispielsweise unter Nutzung von flüssigen oder gasförmigen Wärmeträgem zur Beheizung oder Kühlung der Wände zu verwenden. Somit lassen sich optimale Oberflächentemperaturen einstellen, um beispielsweise Produktablagerungen zu vermeiden.

Im Prozessraum 8 oder in den darüberliegenden Apparateteilen, der Expansionszone 14 und dem Abluftteil 19, können optional Sprühdüsen 6 angeordnet werden, die vorzugsweise nach unten aber auch teilweise nach oben sprühen. Hier kann ebenfalls die flüssige Formulierung eingedüst werden, um beispielsweise durch Sprühtrocknung / Sprüherstarrung im Apparat Granulationskeime zu erzeugen. Alternativ können über einige der Sprüheinrichtungen 6 und 7 Additive oder andere Komponenten in flüssiger Form eingesprüht und somit in die Granulatstruktur homogen eingebettet werden. Wenn die Sprühdüsen 7 die temperaturbeaufschlagte Zuluftkammer 17 passieren, können optional die flüssigkeitsführenden Teile mit Isolationen oder unterschiedlichen Kühl- oder Heizsystemen 12 versehen werden, um Schädigungen an der flüssigen Formulierung zu unterbinden.

Als weiterer Vorteil des erfindungsgemäßen Prozesses ist der sehr einfache Aufbau zu nennen, der eine hohe Betriebssicherheit und Störungsunempfindlichkeit mit sehr guter Reinigungsmöglichkeit verbindet. Somit werden verbesserte Produktionsbedingungen insbesondere hinsichtlich der Pharma - und Hygieneanforderungen bei Produktwechsel geschaffen.

### Beispiele

Die Erfindung wird anhand konkreter Anwendungsbeispiele veranschaulicht, ohne dadurch in irgendeiner Weise eingeschränkt zu werden.

### Beispiel 1

Es wurde eine Ibuprofenschmelze mit einer Temperatur von 110°C in einen Apparat eingesprüht, der durch den zuvor beschriebenen Aufbau gekennzeichnet ist. Der Prozessraum ist gekennzeichnet durch einen rechteckigen Querschnitt und hat oberhalb der geneigten Seitenwände eine Querschnittsfläche von 0,15 x 0,2 = 0,03 m² und eine Höhe von etwa 1 m. Die Zufuhr des auf etwa 40°C vorgeheizten Prozessluftstromes von etwa 150 m³/h erfolgte über 2 längs durch den Apparat verlaufende Gaszuführungsspalte. Die Schmelze wurde über eine druckluftbeaufschlagte vertikal nach oben sprühende Zweistoffdüse in den Prozessluftstrahl mit einem Massenstrom von etwa 30 g/min eingesprüht. Die Verdüsungsluft wurde auf 90°C temperiert. Im Prozessraum befanden sich etwa 500 g an Ibuprofen - Granulaten. Die Entstaubung der Abluft erfolgte durch einen dem Apparat nachgeschalteten Zyklon, und der abgeschiedene Feststoff wurde gravimetrisch in den Prozessraum in Spaltnähe als Keimematerial zugeführt. Die kontinuierliche Entnahme von Granulaten aus dem Prozessraum erfolgte stirnseitig unter Verwendung eines Zick-Zack-Sichters. Die im Sichter abgetrennten Feinanteile wurden durch die Sichtluft in den Prozessraum zurückgeblasen. Das entnommene Granulat hat eine übliche unverfestigte Schüttdichte sowie eine übliche Korngrößenverteilung und ist somit für die Weiterverarbeitung geeignet. So ergab sich die folgende Korngrößenverteilung (Siebanalyse):
> 400 µm : 0,8 Mass.-%
315...400 um : 6,8 Mass.-%
250...315 µm : 15,3 Mass.-%
160...250 µm : 42,3 Mass.-%
100...160 µm : 24,9 Mass.-%
0...100 µm : 9,9 Mass.-%

### Beispiel 2

Es wurde eine Ibuprofenschmelze mit einer Temperatur von 110°C in einen Apparat eingesprüht, der durch den zuvor beschriebenen Aufbau gekennzeichnet ist. Der Prozessraum ist gekennzeichnet durch einen rechteckigen Querschnitt und hat oberhalb der geneigten Seitenwände eine Querschnittsfläche von 0,2x1,0=0,2 m² und eine Höhe von etwa 1 m. Die Zufuhr des auf etwa 45°C vorgeheizten Prozessluftstromes von etwa 780 m³/h erfolgte über 2 längs durch den Apparat verlaufende Gaszuführungsspalte. Der gesamte Prozessluftstrom wurde auf 4 gleichgroße Zuluftkammern gleichmäßig verteilt. Der zuvor beschriebene Prozessraum erstreckt sich oberhalb längs der Zuluftkammern und ist nicht unterteilt. Die Schmelze wurde über zwei druckluftbeaufschlagte vertikal nach oben sprühende Zweistoffdüsen in den Prozessluftstrahl mit einem Gesamt-Massenstrom von etwa 22 kg/h eingesprüht. Im Prozessraum befanden sich etwa 6 kg an Ibuprofen-Granulaten. Die Entstaubung der Abluft erfolgte durch einen im Apparat integrierten Patronenfilter, der durch Druckluftimpulse zyklisch abgereinigt wurde. Die unklassierte Entnahme von Granulaten aus dem Prozessraum erfolgte stirnseitig unter Verwendung einer Zellenradschleuse. Anschließend wurde der gesamte ausgetragene Granulatstrom auf eine Siebanlage geleitet, wo ein Überkornanteil (> 400 µm) und ein Unterkornanteil (< 200 µm) abgesiebt wurden. Die im Sieb abgetrennten Unterkornanteile wurden pneumatisch in den Prozessraum zurückgeblasen. Das Überkorn wurde kontinuierlich in einer Stiftmühle zermahlen und ebenfalls als Granulationskeime in den Prozessraum zurückgefördert. Das entnommene Granulat (> 200 µm und < 400 µm) hat auch hier eine für die Weiterverarbeitung geeignete ausreichende unverfestigte Schüttdichte sowie ausreichende Korngrößenverteilung, welche nachfolgend aufgeführt ist:
> 400 µm : 0,8 Mass.-%
315...400 µm : 6,8 Mass.-%
250...315 µm : 15,3 Mass.-%
160...250 µm : 42,3 Mass.-%
100...160 µm : 24,9 Mass.-%
0...100 µm : 9,9 Mass.-%

### Bezugszeichen

| | |
|---|---|
| 1 | Spaltöffnung(en) |
| 2 | Gasstrahl(en) |
| 3 | Umlenkteil |
| 4 | Austragsorgan |
| 5 | Seitenwand |
| 6 | Sprühdüse(n) in beliebige Richtungen sprühend |
| 7 | Sprühdüse(n) aufwärts sprühend |
| 8 | Prozessraum |
| 9 | Querschnitt einer Prozessstufe |
| 10 | Prozessgas |
| 11 | Abgas |
| 12 | Isolation mit Kühl- oder Heizsystem |
| 13 | Eintragssystem |
| 14 | Expansionszone |
| 15 | Feststoffzirkulation |
| 16 | Leitblech(e) |
| 17 | Zuluftkammer |
| 18 | Druckluftimpulse |
| 19 | Abluftteil |
| 20 | feststoffbeladene Abluft |
| 21 | abgetrennter und zurückgeführter Feststoff |
| 22 | Bedüsungszone |
| 23 | Partikelaustrittes aus dem Gasstrahl |
| 24 | Rücklaufzone |
| 25 | Entstaubungsanlage |
| 26 | Zuführungen |
| 27 | Mechanische Aggregate zur Zerkleinerung |
| 28 | Wärmeübertragungseinrichtungen |
| 29 | Seitenwand |

## Patentansprüche

1. Verfahren zur Herstellung von Teilchen mit einem Länge-Breite-Verhältnis von weniger als 1,4, speziell weniger als 1,3, vorzugsweise weniger als 1,2, noch stärker bevorzugt weniger als 1,1 und insbesondere weniger als 1,05, aus einer pharmazeutischen Substanz, das folgende Stufen umfasst:
(a) Bereitstellung einer Schmelze der pharmazeutischen Substanz;
(b) Erzeugen von Tröpfchen der Schmelze durch Einsprühen in einen Prozessraum;
(c) wiederholtes Vorbeiführen von festen Teilchen an eingesprühten Tröpfchen in dem Prozessraum mit Hilfe eines definiert geführten Prozessgasstrahls, dessen Temperatur in Abhängigkeit vom Erstarrungspunkt der Schmelze festgelegt ist, so dass zumindest ein Teil der Tröpfchen mit Teilchen in Kontakt kommt und auf diesen erstarrt;
(d) Entnehmen von Teilchen aus dem Prozessraum in Abhängigkeit von der Teilchengröße.

2. Verfahren nach Anspruch 1, wobei die mittlere Teilchengröße der hergestellten Teilchen im Bereich von 0,1 bis 3 mm liegt.

3. Verfahren nach einem der Ansprüche 1 oder 2, wobei in dem Prozessraum eine Strahlschicht aus fluidisierten festen Teilchen ausgebildet ist, wobei vorzugsweise das Vorbeiführen von festen Teilchen an eingesprühten Tröpfchen erfolgt, indem Tröpfchen der Schmelze in die Strahlschicht eingesprüht werden.

4. Verfahren nach Anspruch 3, wobei die Tröpfchen der Schmelze im Bereich der Auswurfzone in die Strahlschicht eingesprüht werden.

5. Verfahren nach Anspruch 4, wobei die Tröpfchen gleichgerichtet zum Prozessgasstrahl eingesprüht werden.

6. Verfahren nach Anspruch 4 oder 5, wobei die Tröpfchen in die Auswurfzone in dem Bereich eingesprüht werden, der an die Rücklaufzone angrenzt.

7. Verfahren nach einem der Ansprüche 1 bis 6, wobei die Temperatur des definiert geführten Prozessgasstrahls 10°C bis 40°C unterhalb des Erstarrungspunktes der Schmelze liegt.

8. Verfahren nach einem der Ansprüche 1 bis 7, wobei die Schmelze eine homogene flüssige Phase darstellt oder wobei die Schmelze darin dispergierte feste Stoffe enthält.

9. Verfahren nach einem der Ansprüche 1 bis 8, wobei in den Prozessraum von außen feste Teilchen eingeführt werden.

10. Verfahren nach einem der Ansprüche 1 bis 9, wobei es sich bei der pharmazeutischen Substanz um einen Arzneimittelwirkstoff, vorzugsweise Ibuprofen, handelt.

11. Teilchen hergestellt nach einem Verfahren nach einem der Ansprüche 1 bis 10.

12. Kollektiv von Teilchen aus einer pharmazeutischen Substanz, wobei die Teilchen eine mittlere Teilchengröße von 0,1 bis 3 mm aufweisen und wobei mindestens 90 % der Teilchen ein Länge-Breite-Verhältnis von weniger als 1,4 aufweisen und aus einer Schmelze der pharmazeutischen Substanz hergestellt sind.

13. Kollektiv von Teilchen gemäß Anspruch 12, wobei die pharmazeutische Substanz Ibuprofen ist.

14. Kollektiv von Teilchen gemäß Anspruch 12, hergestellt nach einem Verfahren nach einem der Ansprüche 1 bis 10.

15. Verwendung von Teilchen gemäß der Definition in Anspruch 11 oder eines Kollektivs nach einem der Ansprüche 12 bis 14 zur Herstellung einer pharmazeutischen Darreichungsform.

## Claims

1. Method for producing particles with a length-width ratio of less than 1.4, in particular of less than 1.3, preferably of less than 1.2, still more preferably of less than 1.1 and particular preferred of less than 1.05, from a pharmaceutical substance, which method comprises the following stages:
(a) provision of a melt of the pharmaceutical substance;
(b) production of droplets of the melt by spraying into a processing chamber;
(c) repeated guiding of solid particles past sprayed droplets in the processing chamber with the aid of a process gas jet which is guided in a defined way and whose temperature is fixed, depending on the solidification point of the melt, so that at least some of the droplets come into contact with particles and solidify thereon;
(d) removal of particles from the processing chamber as a function of the particle size.

2. Method according to claim 1, where the average particle size of the produced particles is in the range from 0.1 to 3 mm.

3. Method according to anyone of claims 1 or 2, where a spouted bed of fluidized solid particles is formed in the processing chamber, wherein preferably guiding of solid particles past sprayed droplets takes place by spraying droplets of the melt into the spouted bed.

4. Method according to claim 3, where the droplets of the melt are sprayed into the spouted bed in the region of the ejection zone.

5. Method according to claim 4, where the droplets are sprayed in the same direction as the process gas jet.

6. Method according to anyone of claims 4 or 5, where the droplets are sprayed into the ejection zone in the region adjoining the return zone.

7. Method according to anyone of claims 1 to 6, where the temperature of the process gas jet which is guided in a defined way is 10 °C to 40 °C below the solidification point of the melt.

8. Method according to anyone of claims 1 to 7, where the melt represents a homogeneous liquid phase and where the melt comprises solid substances dispersed therein.

9. Method according to anyone of claims 1 to 8, where solid particles are introduced into the processing chamber from outside.

10. Method according to anyone of claims 1 to 9, where the pharmaceutical substance is an active pharmaceutical ingredient, preferably ibuprofen.

11. Particles produced by a method according to anyone of claims 1 to 10.

12. Collection of particles of a pharmaceutical substance, where the particles have an average particle size of from 0.1 to 3 mm, and where at least 90% of the particles have a length-width ratio of less than 1.4, and are produced from a melt of the pharmaceutical substance.

13. Collection of particles according to claim 12, where the pharmaceutical substance is ibuprofen.

14. Collection of particles according to claim 12, produced by a method according to anyone of claims 1 to 10.

15. Use of particles as defined in claim 11 or of a collection according to anyone of claims 12 to 14 for producing a pharmaceutical dosage form.

## Revendications

1. Procédé pour la préparation de particules avec un rapport longueur sur largeur de moins de 1,4, en particulier de moins de 1,3, préférablement de moins de 1,2, plus particulièrement préféré de moins de 1,1, et surtout de moins de 1,05 à partir d'une substance pharmaceutique lequel comprend les étapes suivantes :
(a) Mise à disposition d'une fusion de la substance pharmaceutique ;
(b) Génération de gouttelettes de la fusion par vaporisation dans un compartiment de procédure ;
(c) Passage répétitif de particules solides a des gouttelettes vaporisés dans le compartiment de procédure à l'aide d'un jet de gaz de procédure conduit de manière définie, la température duquel étant préfixé en dépendance du point de solidification de la fusion, afin qu'au moins une partie des gouttelettes entre en contact avec des particules et se solidifie sur ces-mêmes ;
(d) Extraction de particules du compartiment de procédure en dépendance de la taille de particule.

2. Procédé selon la revendication 1, dans lequel la taille de particule moyenne des particules générées se situe dans la région de 0,1 à 3 mm.

3. Procédé selon l'une quelconque des revendications 1 ou 2, dans lequel une couche de jet de particules solides fluidifiées est formée dans le compartiment de procédure, le passage de particules solides a des gouttelettes vaporisées s'effectuant préférablement en ce que des gouttelettes de la fusion sont vaporisés dans la couche de jet.

4. Procédé selon la revendication 3, dans lequel les gouttelettes de la fusion sont vaporisées dans la couche de jet dans la partie de la zone d'éjection.

5. Procédé selon la revendication 4, dans lequel les gouttelettes sont vaporisées en même direction avec le jet de gaz de procédure.

6. Procédé selon l'une quelconque des revendications 4 ou 5, dans lequel les gouttelettes sont vaporisés dans la partie de la zone d'éjection vicinale a la zone de RÜCKLAUF.

7. Procédé selon l'une quelconque des revendications 1 à 6, dans lequel la température du jet de gaz de procédure conduit de manière définie est entre °C et 40°C en dessous du point de solidification de la fusion.

8. Procédé selon l'une quelconque des revendications 1 à 7, dans lequel la fusion représente une phase homogène liquide, ou dans lequel la fusion comprend des substances solides dispersées en elle.

9. Procédé selon l'une quelconque des revendications 1 à 8, dans lequel des particules solides sont introduites de l'extérieur dans le compartiment de procédure.

10. Procédé selon l'une quelconque des revendications 1 à 9, la substance pharmaceutique étant uns substance pharmaceutiquement active, préférablement l'ibuprofène.

11. Particule préparée selon un procédé selon l'une des revendications 1 à 10.

12. Collectif de particules d'une substance pharmaceutique, les particules ayant une taille moyenne d'entre 0,1 et 3 mm et au moins 90% des particules ayant un rapport longueur sur largeur de moins qu'environ 1,4 et étant préparé à partir d'une fusion de la substance pharmaceutique.

13. Collectif de particules selon la revendication 12, la substance pharmaceutique étant l'ibuprofène.

14. Collectif de particules selon la revendication 12, préparé selon un procédé selon l'une des revendications 1 à 10.

15. Usage de particules selon la définition dans la revendication 11 ou d'un collectif selon l'une des revendications 12 à 14 pour la fabrication d'une forme de dosage pharmaceutique.
